# EUROPEAN PATENT APPLICATION

(11) **EP 1 275 655 A1**
(43) Date of publication of application: **15.01.2003**
(21) Application number: 02021105.8
(22) Date of filing: 13.07.1999
(51) Int. Cl.: C07H 21/00, C12Q 1/68

(54) **Silicon-containing linkers for nucleic acid mass markers**

(30) Priority: 13.07.1998 GB 9815164
(62) Divisional of application: 99933026.9
(71) Applicant: Xzillion GmbH & CO.KG, 65926 Frankfurt am Main (DE)
(72) Inventor: Thompson, Andrew Hugin, Cambridge CB4 3QF (GB); Schmidt, Gunter, Houghton, Cambs PE17 2BQ (GB); Johnstone, Robert Alexander Walker, Bebington L63 9LD (GB)
(74) Representative: Hill, Christopher Michael

(57) **Abstract**

Provided is a method for characterising an analyte, which method comprises:
(a) providing a compound in which the analyte is attached by a cleavable linker to a reporter group relatable to the analyte, the linker having the following formula: 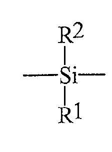 wherein R¹ and R² are substituents as defined in any of claims 1-4 16 and 17;
(b) cleaving the reporter group from the analyte; and
(c) identifying the reporter group, thereby characterising the analyte.

## Description

This invention concerns compounds which comprise mass markers for detection by mass spectrometry. The invention relates to methods for characterising nucleic acids or other molecules by labelling with markers that are cleavably detachable from their associated nucleic acid and that are detectable by mass spectrometry. Specifically this invention relates to improved methods of detaching mass labels from their associated nucleic acids or other molecules of interest.

PCT/GB98/00127 describes arrays of cleavable labels that are detectable by mass spectrometry which identify the sequence of a covalently linked nucleic acid probe. These mass labels have a number of advantages over other methods of analysing nucleic acids. At present commercially favoured systems are based on fluorescent labelling of DNA. Fluorescent labelling schemes permit the labelling of a relatively small number of molecules simultaneously, typically 4 labels can be used simultaneously and possibly up to eight. However the costs of the detection apparatus and the difficulties of analysing the resultant signals limit the number of labels that can be used simultaneously in a fluorescence detection scheme. An advantage of using mass labels is the possibility of generating large numbers of labels which have discrete peaks in a mass spectrum allowing similar numbers of distinct molecular species to be labelled simultaneously. Fluorescent dyes are expensive to synthesise whereas mass labels can comprise relatively simple polymers permitting combinatorial synthesis of large numbers of labels at low cost.

A feature of the mass labelling techniques disclosed in PCT/GB98/00127 is the need for linker groups that covalently link a mass marker to its corresponding nucleic acid. These linkers must permit the mass marker to be separated from its nucleic acid prior to detection within a mass spectrometer. It is desirable that the cleavage of the label from its nucleic acid be performed in-line with a mass spectrometer, possibly after some in-line pre-fractionation step such as capillary electrophoresis. It is also desirable that this in-line cleavage step does not require a complex interface with the mass spectrometer to enable this step to occur. Ideally linkers should cleave at some predetermined point within existing instruments without any modification to the instrument beyond changes of normal operating parameters.

Linkers should cleave without damaging associated nucleic acids hence reducing noise in the mass spectrum from nucleic acid fragmentation. Linkers should all cleave under the same conditions to ensure all labels can be analysed simultaneously and quantitatively.

It is an object of this invention to provide linkers that have the desired features disclosed above which are compatible with existing mass spectrometers particularly electrospray ionisation and tandem mass spectrometry

Accordingly, the present invention provides a compound having the following formula (I): 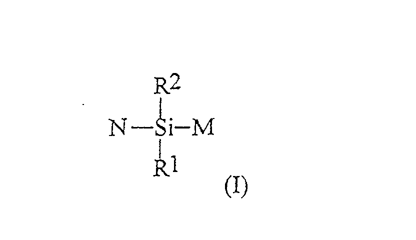 wherein M comprises a mass marker, N comprises a nucleic acid and R¹ and R² are substituents selected such that when the compound reacts with an electron donating moiety, either N or M cleaves from the Si atom in preference to R¹ and R².

Compounds with the formula (I) shown above meet the specification discussed above. The molecule is stable during synthesis and can be cleaved under mild conditions in an electrospray ion source or in the collision chamber of a tandem mass spectrometer in the presence of an appropriately reactive gaseous electron donating moiety, such as ammonia. The reactive gas participates in a novel gas phase reaction with the linker resulting in the cleavage of the linker.

The present invention also provides a method for characterising an analyte, which method comprises:
(a) providing a compound in which the analyte is attached by a cleavable linker to a reporter group relatable to the analyte, the linker having the following formula: 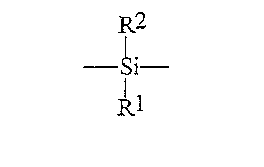 wherein R¹ and R² are substituents as defined below;
(b) cleaving the reporter group from the analyte; and
(c) identifying the reporter group, thereby characterising the analyte.

The invention additionally provides use of a linker group in the characterisation of an analyte, to attach a reporter group to the analyte, wherein the linker group is cleavable and has the following formula: 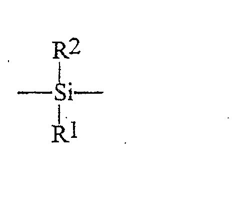 wherein R¹ and R² are substituents as defined below.

The invention will now be described in further detail by way of example only, with reference to the accompanying drawings, in which:

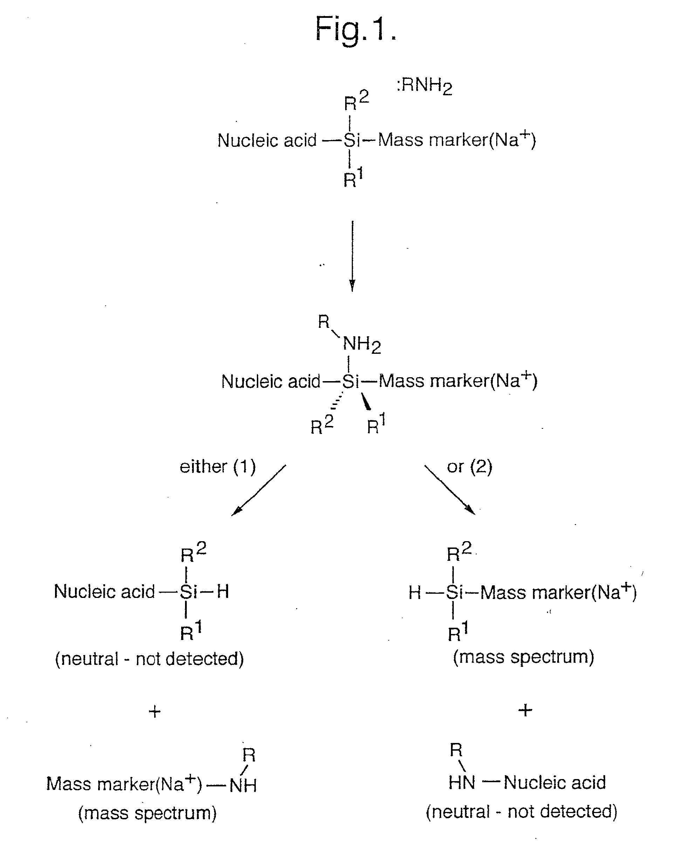

Figure 1 depicts the mechanism of cleavage of a linker used in the present invention, by means of a primary amine. Cleavage takes place via a five-co-ordinate intermediate to produce two possible products. The mass spectrum of the charged products is measured;

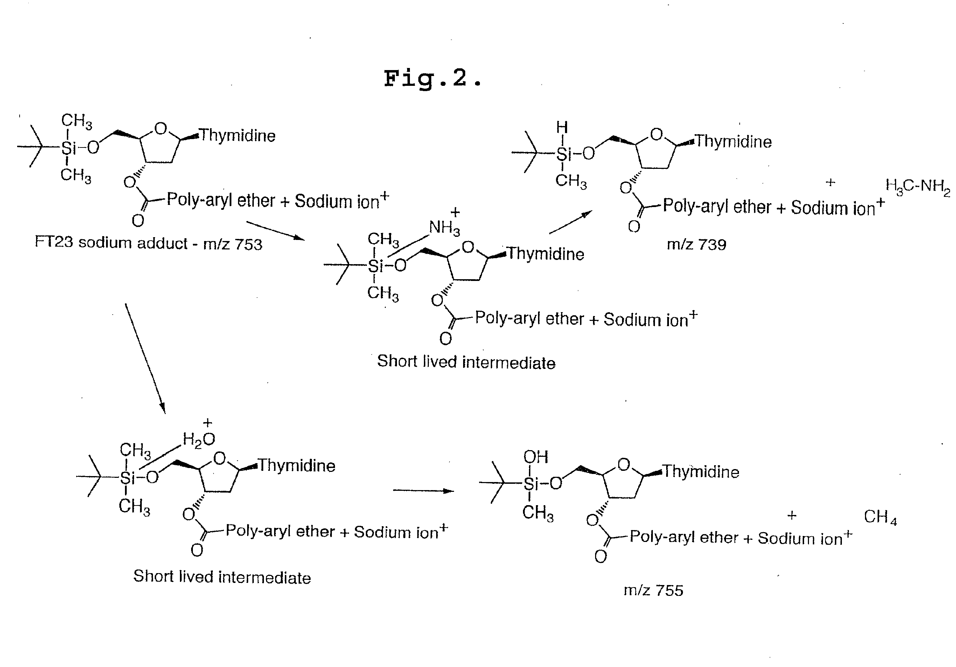

Figure 2 shows a reaction mechanism for ammonia reacting with a TBDMS protective group used in the present invention.

In the methods of the present invention, the analyte is not particularly limited and can be any analyte or molecule of interest, such as a nucleic acid or other molecule. Typically the analyte comprises a biological molecule. In preferred embodiments of the present invention, the biological molecule is selected from a protein, a polypeptide, an amino acid, a nucleic acid (e.g. an RNA, a DNA, a plasmid, a nucleotide or an oligonucleotide), a nucleic acid base, a pharmaceutical agent or drug, a carbohydrate, a lipid, a natural product and a synthetic compound from an encoded chemical library. When the analyte comprises a nucleotide, oligonucleotide or nucleic acid, the nucleotide, oligonucleotide or nucleic acid may be natural, or may be modified by modifying a base, sugar and/or backbone of the nucleotide, oligonucleotide or nucleic acid. In the compounds of the present invention, the analyte is a nucleic acid, and may be any type of nucleic acid. Preferably, the nucleic acid is of a type as defined above.

The substituents R¹ and R² are not especially limited. It is preferred that R¹ and R² are selected such that their bond energies to Si are greater than the bond energy of N and/or M to Si to ensure that when the compound is reacted with an electron donating moiety either N or M cleaves from the Si atom in preference to R¹ and R², and/or R¹ and R² are selected such that their steric bulk is sufficient to ensure that when the compound is reacted with an electron donating moiety either N or M cleaves from the Si atom in preference to R¹ and R². Typically, R¹ and R² are each independently a hydrogen atom, a halogen atom, a substituted or unsubstituted alkyl group, or a substituted or unsubstituted aryl group. It is particularly preferred that R¹ and R² are each independently fluorine, chlorine, bromine, iodine, methyl, ethyl, propyl, isopropyl, butyl, isobutyl, tert-butyl or phenyl groups.

Thus, various substituents may be introduced at the positions R¹ and R² including fluorine, chlorine and other halogens, methyl, ethyl and other alkyl groups. Phenyl groups may also be appropriate. Preferably substituents at R¹ and R² should be stable during synthesis of the marker, during incorporation of the mass label into an oligonucleotide in an automated synthesiser and under mass spectrometry. A wide variety of groups have these properties and may be incorporated into the linker at these positions. It may also be desirable in some embodiments to choose substituents which change the solubility of the linker and alter the rigidity of the linker.

Preferably a covalent linkage is formed in attaching the analyte and/or the reporter group to the cleavable linker. The covalent linkage is not particularly limited provided that the analyte and/or reporter group can readily be attached to the cleavable linker using reactive functionalities attached to the linker and the analyte.

Table 1 below lists some reactive functionalities that may be reacted together to generate a covalent linkage between two entities. Any of the functionalities listed below could be used to form the compounds used in the present invention to permit the linker to be attached to an analyte (such as a nucleic acid or protein) for detection (e.g. by mass spectrometry). If desired, a reactive functionality can be used to introduce a further linking group with a further reactive functionality.

**Table 1**

| **Functionality 1** | **Functionality 2** | **Resultant Covalent Linkage** |
|---|---|---|
| -NH₂ | -COOH | -CO-NH- |
| -NH₂ | -NCO | -NH-CO-NH- |
| -NH₂ | -NCS | -NH-CS-NH- |
| -NH₂ | -CHO | -CH₂-NH- |
| -NH₂ | -SO₂Cl | -SO₂-NH- |
| -NH₂ | -CH=CH- | -NH-CH₂-CH₂- |
| -OH | -OP(NCH(CH₃)₂)₂ | -OP(=O)(O)O- |

It should be noted that some of the reactive functionalities above or their resultant covalent linkages might have to be protected prior to introduction into an oligonucleotide synthesiser. Preferably unprotected ether, ester, thioether and thioesters, amine and amide bonds are to be avoided as these are not stable in an oligonucleotide synthesiser. A wide variety of protective groups are known in the art to protect linkages from unwanted side reactions.

A short alkyl linkage is appropriate to link the mass marker to the linker, although a wide variety of linkages are available which can be used to link a mass marker to a linker.

The reporter group used in the present invention is not especially limited and may be any group, provided that it'is readily detectable and can se related to an analyte to identify the analyte. Typically, the reporter group is a mass marker, that is detectable by mass spectrometry. Other appropriate reporters include fluorophores, radiolabels, chemiluminescent labels, and electron capture labels. In the compounds of the present invention, the reporter group comprises a mass marker.

In preferred embodiments of the present invention, mass markers disclosed in PCT/GB98/00127, PCT/GB98/03842, GB 9815166.5 and GB 9826159.7 can be employed. The content of these applications is incorporated by reference. PCT/GB98/00127 and PCT/GB98/03842 disclose poly-ether mass markers which are thermally stable, chemically inert and fragmentation resistant compounds, and which can be substituted with a variety of groups to alter properties such as solubility and charge. These mass markers are also preferred for use in the present invention and the content of this application is incorporated by reference. GB 9826159.7 discloses markers which comprise two components, which may be poly-ethers, which are analysed by selected reaction monitoring. These are particularly preferred mass markers for use in the present invention. GB 9815166.5 discloses mass markers that bind metal ions, which are also preferred markers for use with this invention. The content of this application is incorporated by reference. Reporter groups that can be detected by more than one detection means may also be desirable as with, for example, a fluorescent marker that incorporates a radioisotope in its linker and that is detectable by mass spectrometry and reporters of this kind are refereed to as 'multi-mode reporter' groups. Preferred multi-mode reporter groups are detectable by mass spectrometry.

When the mass marker comprises an oligoether or a polyether, the oligoether or polyether may be a substituted or unsubstituted oligo- or poly-arylether. The oligoether or polyether preferably comprises one or more fluorine atom or methyl group substituents, or one or more ²H or ¹³C isotopic substituents.

It is further preferred that the mass marker comprises a metal ion-binding moiety. Typically, the metal ion-binding moiety comprises a porphyrin, a crown ether, hexahistidine, or a multidentate ligand. Preferably, the metal ion-binding moiety is a bidentate ligand or is EDTA. The metal ion-binding moiety may be bound to a monovalent, divalent or trivalent metal ion. The metal ion is not especially limited. Preferred metal ions include a transition metal ion, or a metal ion of group IA, IIA or IIIA of the periodic table. Particularly preferred metal ions are Ni²⁺, Li⁺, Na⁺, K⁺, Mg²⁺, Ca²⁺, Sr²⁺, Ba²⁺, or Al³⁺. The presence of a metal ion on the mass marker increases the sensitivity of detection.

It should be noted that this invention is not limited to the mass markers disclosed in the above applications. Any molecule with the correct features can be used as a mass marker. Desirable features include:
- Easily detachable from DNA
- Fragmentation resistant in mass spectrometer
- Single ion peaks
- Very sensitive detection
- Easily distinguishable from background contamination
- Distinguish from DNA
- Be certain that a mass peak is from a mass label
- Compatible with oligonucleotide synthesiser
- Easy to synthesise in a combinatorial manner to minimise number of chemical steps and the number of reagents necessary to generate large number of labels
- Compatible with existing mass spectrometry instrumentation without requiring physical modification.

Mass labels and their linkers can be attached to a nucleic acid molecule at a number of locations in the nucleic acid. For conventional solid phase synthesisers the 5' hydroxyl of the sugar is the most readily accessible. Other favoured positions for modifications are on the base at the 5' position in the pyrimidines and the 7' and 8' positions in the purines. These would all be appropriate positions to attach a cleavable mass with the linker of this invention.

The 2' position on the sugar is accessible for mass modifications but is more appropriate for small mass modifications that are not to be removed.

The phosphate linkage in natural nucleic acids can be modified to a considerable degree as well, including derivitisation with mass labels.

The cleavable linker used in this invention may be cleaved in the ion source of a mass spectrometer by ammonia. However, this invention is not limited to the use of ammonia. Most amines are be capable of separating the mass marker from its cognate oligonucleotide and other nucleophiles may also be used.

## Claims

1. A compound having the following formula (I): 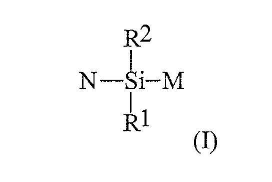 wherein M comprises a mass marker, N comprises a nucleic acid, and wherein R¹ and R² are each independently selected from a hydrogen atom, a halogen atom, a substituted or unsubstituted alkyl group, and a substituted or unsubstituted aryl group such that when the compound reacts with an electron donating moiety, either N or M cleaves from the Si atom in preference to R¹ and R².

2. A compound according to claim 1, wherein R¹ and R² are each independently selected from fluorine, chlorine, bromine, iodine, methyl, ethyl, propyl, isopropyl, butyl, isobutyl, tert-butyl or phenyl groups.

3. A compound according to claim 1 or claim 2, wherein N comprises a nucleotide or an oligonucleotide.

4. A compound according to claim 3, wherein the nucleotide or oligonucleotide is natural, or is modified by modifying a base, sugar and/or backbone of the nucleotide or oligonucleotide.

5. A compound according to any preceding claim, wherein the mass marker comprises a polyether.

6. A compound according to claim 5, wherein the polyether is a substituted or unsubstituted poly(arylether).

7. A compound according to claim 5 or claim 6, wherein the polyether comprises one or more fluorine atom substituents.

8. A compound according to any preceding claim, wherein the mass marker comprises a metal ion-binding moiety.

9. A compound according to claim 8, wherein the metal ion-binding moiety is a porphyrin, a crown ether, hexahistidine, or a multidentate ligand.

10. A compound according to claim 9, wherein the metal ion-binding moiety is a bidentate ligand or is EDTA.

11. A compound according to any of claims 8-10, wherein the metal ion-binding moiety is bound to a monovalent, divalent or trivalent metal ion.

12. A compound according to claim 11, wherein the metal ion is a transition metal ion, or a metal ion of group IA, IIA or IIIA of the periodic table.

13. A compound according to claim 12, wherein the metal ion is Ni²⁺, Li⁺, Na⁺, K⁺, Mg²⁺, Ca²⁺, Sr²⁺, Ba²⁺, or Al³⁺.

14. A compound according to any preceding claim, wherein the electron donating moiety is a Lewis base.

15. A compound according to claim 14, wherein the Lewis base is selected from ammonia; a primary, secondary or tertiary amine; a compound containing a hydroxy group; an ether; and water.

16. A method for characterising an analyte, which method comprises:
(a) providing a compound in which the analyte is attached by a cleavable linker to a mass marker identifiable by mass spectrometry and relatable to the analyte, the linker having the following formula: 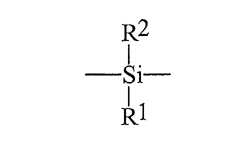 wherein R¹ and R² are substituents as defined in any of claims 1, 2, 14 and 15;
(b) cleaving the mass marker from the analyte in a mass spectrometer; and
(c) identifying the mass marker by mass spectrometry, thereby characterising the analyte.

17. A method according to claim 16, wherein the mass marker is as defined in any of claims 5-13.

18. A method according to claim 16 or claim 17, wherein the analyte is a nucleic acid.

19. A method according to claim 18, wherein the nucleic acid is as defined in claim 3 or claim 4.

20. A method according to any of claims 16-19, which method further comprises forming a compound as defined in any of claims 1-15, prior to identifying the reporter group.

21. A method according to any of claims 16-20, which method further comprises contacting the linker with an electron donating moiety to cleave off the mass marker.

22. A method according to claim 21, wherein the electron donating moiety is as defined in claim 14 or claim 15.

23. Use of a linker group in the characterisation of an analyte, to attach a mass marker identifiable by mass spectrometry to the analyte, wherein the linker group is cleavable and has the following formula: 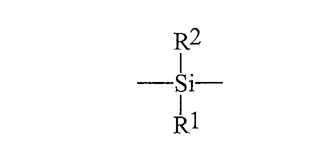 wherein R¹ and R² are substituents as defined in any of claims 1, 2, 14 and 15.

24. Use according to claim 23, wherein the mass marker is as defined in any of claims 5-13.

25. Use according to claim 23 or claim 24, wherein the analyte is a nucleic acid.

26. Use according to claim 25, wherein the nucleic acid is as defined in claim 3 or claim 4.

27. Use according to any of claims 23-26, wherein the mass marker forms part of a compound as defined in any of claims 1-15.
